(19)

Europäisches
Patentamt

European
Patent Office

Office européen
des brevets

(11)  **EP 3 147 366 A1**

(12)  **EUROPEAN PATENT APPLICATION**

(43) Date of publication:
    **29.03.2017  Bulletin 2017/13**

(51) Int Cl.:
    **C12N 15/65** [(2006.01)]    **C07K 14/705** [(2006.01)]
    **C07K 14/47** [(2006.01)]

(21) Application number: **15186957.5**

(22) Date of filing: **25.09.2015**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **MA**

(71) Applicants:
    • **Deutsches Krebsforschungszentrum Stiftung
      des
      Öffentlichen Rechts
      69120 Heidelberg (DE)**
    • **Ruprecht-Karls-Universität Heidelberg
      69117 Heidelberg (DE)**

(72) Inventors:
    • **Beaudouin, Joel
      69115 Heidelberg (DE)**
    • **Liesche, Clarissa
      69115 Heidelberg (DE)**
    • **Eils, Roland
      69198 Schriesheim (DE)**

(74) Representative: **Krauss, Jan
      Boehmert & Boehmert
      Anwaltspartnerschaft mbB
      Patentanwälte Rechtsanwälte
      Pettenkoferstrasse 20-22
      80336 München (DE)**

(54)  **DEATH RECEPTOR BASED SELECTION MARKER**

(57)    The present invention discloses the use of a dominant negative mutant death receptor as a selection marker for cell transfection. The invention provides new genetic constructs for the introduction and expression of heterologous genetic sequences in target host cells. The use of a dominant negative mutant death receptor as selection marker to select for positive transfectants allows for an enrichment of transfected cells and thereby significantly enhances efficiency of the transfection protocols of the herein disclosed invention. The inventive selection marker is preferably applied in genome editing approaches such as clustered, regularly interspaced, short palindromic repeat (CRISPR) mediated genome manipulation.

**EP 3 147 366 A1**

**Description**

FIELD OF THE INVENTION

The present invention discloses the use of a dominant negative mutant death receptor as a selection marker for cell transfection. The invention provides new genetic constructs for the introduction and expression of heterologous genetic sequences in target host cells. The use of a dominant negative mutant death receptor as selection marker to select for positive transfectants allows for an enrichment of transfected cells and thereby significantly enhances efficiency of the transfection protocols of the herein disclosed invention. The inventive selection marker is preferably applied in genome editing approaches such as clustered, regularly interspaced, short palindromic repeat (CRISPR) mediated genome manipulation.

DESCRIPTION

The clustered, regularly interspaced, short palindromic repeat (CRISPR) technology has become an extremely powerful tool to edit eukaryotic genes. This technology has been adapted from a bacterial defense system against foreign DNA and only requires a nuclease that recognizes a designed, single guide RNA (gRNA) that targets a DNA sequence of interest. The outcome is the induction of a double-stranded DNA break at the target site. Repeated cycles of cut and faulty repair by the cellular non-homologous end joining machinery can eventually generate insertion/deletion (indel) mutations, and consequently a knock-out effect in cases where these mutations introduce a frame-shift within the targeted gene.

By targeting DNA, the CRISPR/Cas9 system perfectly complements gene knock-down on the RNA level by RNA interference for the annotation of gene functions. Importantly, as compared to siRNA/shRNA technology, phenotyping by gene knock-out is not constrained by a reduction of gene expression that can be incomplete and that can vary from cell to cell. Still, the efficiency of gene knock-out within a cell population remains a limiting and highly variable factor. For example, the first studies validating the CRISPR approach for mammalian cells demonstrated proportions of gene-edited cellular subpopulations ranging between 2% and 25%, depending on the cell type and the mode of Cas9/gRNA transfection. Two main strategies have improved the efficiency, the first being the optimization of the delivery method, using e.g. lentiviral, adenoviral or Adeno-associated viral (AAV) vectors. Lentivirus libraries have successfully been used for various screening projects and AAVs have been applied in animals. Potential drawbacks of this viral vector strategy are the requirement to work under elevated biosafety levels (lenti- and adenoviruses), the stable chromosomal integration of the viral genomes encoding the Cas9 nuclease (lentiviruses), or the limited capacity for packaging of foreign DNA such as Cas9 cDNAs (AAV). The other strategy consists of enriching and expanding from a large population a small pool of cells that transiently express high amounts of Cas9 nuclease together with a selection marker. The use of fluorescent proteins allows for positive selection by flow cytometry sorting, while drug resistance genes permit to enrich desired cells by killing Cas9-negative cells with a specific drug. The cell killing should occur within two days, during the typical peak of expression of the resistance gene. For this reason, puromycin is typically preferred to other drugs as it generally kills cells within 24 to 48 hours. Strategies involving transient expression of Cas9 have the advantage of being scarless, as the only genome modification that is left after the transient expression is the editing event itself, but no Cas9/gRNA insertion or acquired drug resistance. Providing a good balance between killing of non-transfected cells and survival of transfected cells, one can yield efficient genome editing even in the case of suboptimal transfection with limited cost and material.

Apoptosis or programmed cell death is known to be involved in developmental processes that lead to growth and maturation in all animals and humans. Normally, apoptosis is strictly regulated by physiological signals that lead to orderly development in various tissues. All cells have the capacity to undergo apoptosis, but in normal conditions, the apoptotic mechanisms are in the "off" mode. Apoptosis of cells can be triggered by extracellular signaling molecules via the death receptor signaling pathway.

This pathway can be triggered by some members of the tumor necrosis factor (TNF) superfamily, including TNF related apoptosis inducing ligand (TRAIL) and Fas/CD95 ligand (refered as CD95L in the remaining text). Ligation of TRAIL to its cognate receptors can cause cell death by apoptosis or may cause NF-κB activation. There are three known TRAIL isoforms (TRAILα, TRAILβ, and TRAILγ). TRAIL has widespread expression on multiple cell lineages and has shown potent toxicity for many tumors and virally infected cells, while sparing most healthy cells. TRAIL mediates cell death via binding of one of five TRAIL receptors (e.g., TRAIL-R1, -R2, -R3, -R4, and osteoprotegerin (OPG)). TRAIL-R1 and -R2 can cause an apoptotic signal, while TRAIL-R3 and -R4 lack intracellular regions that can propagate an apoptotic signal. The activation of the TNF receptor family members Fas/CD95 (referred as CD95 in the remaining text) by CD95L, or DR4 and DR5 by TRAIL, can induce cell death in many cellular contexts through the activation of caspases. This activation occurs through the recruitment of the adaptor protein FADD on the death domain (DD) of oligomerized, ligand-associated receptors. Because of this oligomerization step, receptor mutants that lack the DD can block apoptosis, even in the presence of wild type (wt) receptors, in a dominant-negative manner.

Genome editing using the prokaryotic CRISPR/Cas9 system has become a major tool for the targeted introduction of genomic manipulations such as deletions and insertions. The method allows for an efficient introduction of mutations into cellular genomes without altering the target genome more than necessary. However, genome editing efficiency is still dependent on the rate of transfection of the CRISPR constructs into the target cells. Thus, it was an object of the present invention to improve efficiency of genome editing protocols by increasing the transfection success. Another object of the present invention was to provide an alternative method for selecting positive transfectants which compared to the use of antibiotics is more efficient and convenient.

The above problem is solved in a first aspect by genetic construct for transfection of a target cell, comprising an expression cassette and a selection marker, characterized in that the selection marker is an expressible sequence encoding for a dominant negative mutant death receptor.

The present invention is based on the idea that in order to select cells that were positively transfected with a genetic construct, the genetic construct contains as a selection marker a dominant negative death receptor mutant. By introducing the dominant negative death receptor mutant into a cell which expresses the wild-type complement of the death receptor, the dominant negative death receptor mutant will inhibit ligand induced death receptor signaling in the transfected cells due to its dominant negative impact on the wild type receptor. Cells that do not contain the genetic construct, and therefore also do not contain the dominant negative death receptor mutant as selection marker of the invention, will be still sensitive for ligand induced death receptor signaling. Therefore, upon contacting cells that underwent a transfection procedure with the genetic construct of the invention with a death receptor ligand results in the activation of death receptor signaling in cells which do not contain the selection marker of the invention, which leads to cell death. The selection marker of the invention therefore allows for a targeted killing of negative transfectants and therefore for an enrichment of positively transfected cells. The only requirement for the use of the selection marker of the invention is that the cells to be transfected contain an active (e.g. wild-type) death receptor which is expressed in the cell and responsive to death receptor ligand contacting. The dominant negative death receptor mutant is then selected to be able to dominant negatively influence the death receptor in the cells to be transfected.

As used herein, the term "genetic construct" is meant to refer to an isolated artificially designed nucleic acid molecule, e.g. DNA, RNA or variants thereof, that comprises a nucleic acid sequence carrying genetic information. The genetic sequence may for example comprise a sequence that encodes a protein or an RNA molecule, and which may preferably be operably linked on the genetic construct to elements necessary for the expression of the nucleic acid sequence such as promoters. In some embodiments, the genetic construct is a plasmid or genome of a viral vector. In some embodiments, the genetic construct is RNA, preferably a genome of a retroviral vector. Further embodiments that are preferred are described herein below.

The term "transfection" as used in context of the present invention shall pertain to a process involving the artificial introduction of an extracellular genetic material or genetic construct, with or without accompanying material such as viral envelopes, into a target cell. The genetic material remains as an extrachromosomal element or is integrated into the chromosome of the target cell and is made to be replicable. Therefore, the invention includes both transient and stable transfections of a target cell. However, preferred embodiments of the invention pertain to transient transfection.

The term "target cell" as used in context of the present invention shall refer to any cell into which a nucleic acid, e.g. encoding a heterologous protein or constituting an RNA gene, can be introduced. Preferably the target cell is a cell that can be manipulated by way of transfection and which expresses or is capable to express a death receptor. Therefore, the target cell of the invention is a cell which is intended to be subject of a transfection with a genetic construct as described herein. In particular, a target cell according to the invention is a eukaryotic cell, preferably a vertebrate or mammalian cell such as a mouse, rat or human cell. In preferred embodiments the target cell according to the invention comprises a wild-type complement to the dominant negative death receptor mutant that is used as selection marker in accordance with the invention. The target cell preferably should have the ability to activate apoptosis in response to death receptor ligand mediated activation (binding) of the death receptor signaling pathway.

The term "expression cassette" as used herein shall refer to a nucleic acid element that allows for the introduction of an expressible sequence into the genetic construct of the invention. The expression cassette may therefore be a simple cloning site. In preferred embodiments the expression cassette may further include genetic elements necessary for the expression of a nucleic acid, such as regulatory elements, promoters, and termination signals. These elements are arranged in the expression cassette to be in functional order for the expression of a nucleic acid sequence to be introduced into the expression cassette. Such elements and their arrangement are well known to the skilled artisan.

Thus, in one embodiment, the expression cassette of the invention comprises a cloning site suitable for the integration of a genetic sequence to be expressed by the genetic construct.

A "selection marker" is a nucleic acid that allows cells carrying the selection marker to be specifically selected for or against, in the presence of a corresponding selection agent. A state of the art selection marker is for example an antibiotic resistance gene. This selection marker allows the host cell transformed therewith to be positively selected for in the presence of the corresponding selection agent, e.g. the antibiotic. Further marker genes are described e.g. in WO 92/08796 and WO 94/28143. However, in context of the present invention the selection marker is a dominant negative mutant death receptor. According to the herein disclosed invention the selection agent is a death receptor ligand which is capable of binding to the dominant negative mutant death receptor of the invention.

A preferred embodiment relates to the genetic construct of the invention for use in a method of clustered regularly interspaced short palindromic repeats (CRISPR) mediated gene editing in a target cell. In this embodiment the expression cassette of the genetic construct comprises an expressible sequence encoding for a genome editing nuclease, preferably a genome editing nuclease selected from TALEN, ZNF-nucleases or CRISPR nucleases, or a functional variant thereof.

The term "nuclease," as used herein, refers to an agent, for example, a protein or a small molecule, capable of cleaving a phosphodiester bond connecting nucleotide residues in a nucleic acid molecule. In some embodiments, a nuclease is a protein, e.g., an enzyme that can bind a nucleic acid molecule and cleave a phosphodiester bond connecting nucleotide residues within the nucleic acid molecule. A nuclease may be an endonuclease, cleaving a phosphodiester bonds within a polynucleotide chain, or an exonuclease, cleaving a phosphodiester bond at the end of the polynucleotide chain. In some embodiments, a nuclease is a site-specific nuclease, binding and/or cleaving a specific phosphodiester bond within a specific nucleotide sequence, which is also referred to herein as the "recognition sequence," the "nuclease target site," or the "target site." In some embodiments, a nuclease is a RNA-guided (i.e., RNA-programmable) nuclease or a DNA-guided nuclease (Argonaut), which complexes with (e.g., binds with) an RNA or DNA (e.g., a guide RNA, "gRNA"; or guide DNA respectively) having a sequence that complements a target site, thereby providing the sequence specificity of the nuclease. In some embodiments, a nuclease recognizes a single stranded target site. In other embodiments, a nuclease recognizes a double- stranded target site, for example, a double- stranded DNA target site. The target sites of many naturally occurring nucleases, for example, many naturally occurring DNA restriction nucleases, are well known to those of skill in the art. In many cases, a DNA nuclease, such as EcoRI, HindIII, or BamHI, recognize a palindromic, double- stranded DNA target site of 4 to 10 base pairs in length, and cut each of the two DNA strands at a specific position within the target site. Some endonucleases cut a double- stranded nucleic acid target site symmetrically, i.e., cutting both strands at the same position so that the ends comprise base-paired nucleotides, also referred to herein as blunt ends. Other endonucleases cut a double- stranded nucleic acid target sites asymmetrically, i.e., cutting each strand at a different position so that the ends comprise unpaired nucleotides. Unpaired nucleotides at the end of a double-stranded DNA molecule are also referred to as "overhangs," e.g., as "5 '-overhang" or as "3 '-overhang," depending on whether the unpaired nucleotide(s) form(s) the 5' or the 5' end of the respective DNA strand. Double-stranded DNA molecule ends ending with unpaired nucleotide(s) are also referred to as sticky ends, as they can "stick to" other double-stranded DNA molecule ends comprising complementary unpaired nucleotide(s). A nuclease protein typically comprises a "binding domain" that mediates the interaction of the protein with the nucleic acid substrate, and also, in some cases, specifically binds to a target site, and a "cleavage domain" that catalyzes the cleavage of the phosphodiester bond within the nucleic acid backbone. In some embodiments a nuclease protein can bind and cleave a nucleic acid molecule in a monomeric form, while, in other embodiments, a nuclease protein has to dimerize or multimerize in order to cleave a target nucleic acid molecule. Binding domains and cleavage domains of naturally occurring nucleases, as well as modular binding domains and cleavage domains that can be fused to create nucleases binding specific target sites, are well known to those of skill in the art. For example, the binding domain of RNA-programmable nucleases (e.g., Cas9), or a Cas9 protein having an inactive DNA cleavage domain, can be used as a binding domain (e.g., that binds a gRNA to direct binding to a target site) to specifically bind a desired target site, and fused or conjugated to a cleavage domain, for example, the cleavage domain of FokI, to create an engineered nuclease cleaving the target site.

Most preferred embodiment of the invention may utilize CRISPR associated protein 9 (Cas9), or a homolog/ortholog thereof, as a nuclease of the invention. The person of skill knows that Cas9 forms part of the bacterial defense mechanism against plasmids and therefore there are many Cas9 orthologs in different bacterial species. Also comprised by the invention are functional fragments of a nuclease such as Cas9, so long as there RNA/DNA guided nuclease activity is retained.

In alternative embodiments, the Cas9 nuclease, or similar nuclease, may be mutated to remove the endonuclease activity. In this embodiment, the sequence specific binding ability of the former nuclease is used to target other enzymatic functionalities or proteins in general to a certain sequence on a genome. For example point mutations can be introduced into Cas9 to abolish nuclease activity, resulting in a dead Cas9 (dCas9) that still retains its ability to bind DNA in a gRNA-guided manner (Qi L S, Larson M H, Gilbert L A, Doudna J A, Weissman J S, Arkin A P, Lim W A. Repurposing CRISPR as an RNA-guided platform for sequence-specific control of gene expression. Cell. 2013; 152(5):1173-83. PMID: 23452860). In principle, when fused to another protein or domain, dCas9 can target that protein to virtually any DNA sequence simply by co-expression with an appropriate gRNA. Of course this technology can be applied to other guided nucleases.

Therefore, the present invention shall also encompass the application of modified sequence specific nucleases, such as RNA guided fusion proteins, to bring enzymes and proteins in general to specific sites in a genome programmed by the gRNA. For example a variety of site-specific genome engineering tools beyond nucleases may be used, including transcriptional activators, transcriptional repressors, histone-modifying proteins, integrases, and recombinases. Some of these potential applications have recently been implemented through dCas9 fusions with transcriptional activators to afford RNA-guided transcriptional activators, transcriptional repressors, and chromatin modification enzymes. Simple co-expression of these fusions with a variety of gRNAs results in specific expression of the target genes. These seminal studies have paved the way for the design and construction of readily programmable sequence-specific effectors for the precise manipulation of genomes. Such applications reviewed in Patrick D. Hsu, Eric S. Lander, and Feng Zhang, "Development and Applications of CRISPR-Cas9 for Genome Engineering", Cell. 2014 Jun 5; 157(6): 1262-1278, and the therein recited modified nucleases are comprised in the scope of the present inventions.

The term "RNA/DNA -programmable nuclease," and "RNA/DNA -guided nuclease" are used interchangeably herein and refer to a nuclease that forms a complex with (e.g., binds or associates with) one or more RNA/DNA that is not a target for cleavage. In some embodiments, an RNA/DNA-programmable nuclease, when in a complex with an RNA/DNA, may be referred to as a nuclease:RNA/DNA complex. Typically, the bound RNA(s)/DNA(s) is referred to as a guide RNA/DNA (gRNA, guide DNA). The sequence for the guide RNA or DNA is also in some embodiments part of the genetic construct of the invention, as will be explained in more detail herein below.

In one embodiment the genetic construct of the invention further comprises at least one promoter operatively linked to the expressible sequence encoding for a genome editing nuclease and/or operatively linked to the expressible sequence encoding for a dominant negative mutant death receptor.

The term "promoter" denotes a polynucleotide sequence which allows and controls the transcription of the genes or sequences operably connected therewith. A promoter contains recognition sequences for binding RNA polymerase and the initiation site for transcription (transcription initiation site). In order to express a desired sequence in a certain cell type or a host cell a suitable functional promoter must be chosen. A large number of promoters, including constitutive, inducible and repressible promoters from a variety of different sources, are well known in the art (and identified in databases such as GenBank) and are available as separate elements or elements cloned within polynucleotide sequences from commercial (e.g. depositories such as ATCC as well as other commercial sources) or individual sources. In inducible promoters the activity of the promoter may be increased or reduced in response to a signal. For example, the tetracycline (tet) promoter containing the tetracycline operator sequence (tetO) can be induced by a tetracycline-regulated transactivator protein (tTA). Binding of the tTA to the tetO is inhibited in the presence of tet. Examples for other inducible promoters are jun, fos, metallothionein and heat shock promoters. Of the promoters which are particularly suitable for high expression in eukaryotes, there are for example the ubiquitin/S27a promoter of the hamster, SV 40 early promoter, adenovirus major late promoter, mouse metallothionein-I promoter, the long terminal repeat region of Rous Sarcoma Virus, the early promoter of human Cytomegalovirus (CMV). Examples of other heterologous mammalian promoters are the actin, immunoglobulin or heat shock promoter(s).

In another embodiment of the invention the dominant negative mutant death receptor is a death receptor comprising a mutated and inactive death domain, preferably comprising a death domain deletion (ΔDD).

The term "death domain" refers to a conserved intracellular region found in receptors like CD95 and TNFR-1 that is capable of inducing apoptosis while in the presence of a particular ligand (in this case, CD95L and TNFalpha respectively). Preferred death receptors of the invention are selected from CD95, DR4 or DR5.

The target cell in context of the present invention may be selected from an animal target cell, preferably a *Drosophila,* a *C.elegans,* a zebrafish, or a mammalian target cell, such as a mouse, rat or human cell; preferably the target cell is a cell expressing wild-type version of the death receptor of the genetic construct of the invention. Since the principle of the present invention is based on the dominant negative effect of the death receptor mutant expressed via the genetic construct on the endogenous death receptor pathway in the target cell, the death receptor must be chosen based on which death receptors are present on the target cell, and which will be blocked in the event of a death receptor ligand induced signaling.

In order to allow for an equal expression of the expressible sequence and the dominant negative mutant death receptor a genetic construct of the invention may be designed wherein the expressible sequence encoding for a CRISPR nuclease and the expressible sequence encoding for a dominant negative mutant death receptor are under control of the same promoter. For a 1:1 stoichiometric expression the expressible sequence encoding for a CRISPR nuclease and the expressible sequence encoding for a dominant negative mutant death receptor may be contained within a single open reading frame (ORF) encoding for a fusion protein composed of the CRISPR nuclease and dominant negative mutant death receptor, and wherein the two expressible sequences within the single ORF are separated in frame by a sequence encoding for a protein separation site, such as a proteolytic cleavage site. Such protein separation sites which are useful in context of the herein disclosed invention may be selected from a 2A peptide, preferably selected from the Foot-and-mouth disease virus (F2A), from the equine rhinitis A virus (E2A), from the *Thosea asigna* virus (T2A) and from the porcine teschovirus-1 (P2A). Other technologies that allow for a post-translational separation of fusion proteins may be used as well and shall be encompassed by the present invention.

Of course the present invention also may comprise other bicistronic systems for the expression of multiple proteins. The term "bicistronic system" refers to a region that can be transcribed to produce a transcript for a plurality of reading frames. For example the genetic construct may comprise internal ribosome entry site (IRES) for the translation of multiple reading frames on one transcript.

An alternative embodiment of the present invention then pertains to a genetic construct as described herein before, wherein the expressible sequence encoding for a CRISPR nuclease and the expressible sequence encoding for a dominant negative mutant death receptor are contained within two separate open reading frames (ORF), wherein said two separate ORFs are under the control of one promoter, or alternatively wherein said two separate ORFs are under control of two separate promoters.

As mentioned already above, the genetic construct of the invention in some preferred embodiments should comprise a guide RNA (gRNA) - expression cassette, composed of an invariant gRNA scaffold and a cloning site for insertion of a gRNA sequence. In alternative embodiments, in case the nuclease used in the genetic construct of the invention is a DNA guided DNA nuclease, the respective guide DNA may be provided as a separate DNA molecule, for example may be separately introduced into the cell by transfection or transduction.

gRNAs (or guide DNAs respectively, which will in the following not explicitly mentioned but are included in the following description regarding gRNA) can exist as a complex of two or more RNAs, or as a single RNA molecule. gRNAs that exist as a single RNA molecule may be referred to as single-guide RNAs (sgRNAs), though "gRNA" is used interchangeably to refer to guide RNAs that exist as either single molecules or as a complex of two or more molecules. Typically, gRNAs that exist as single RNA species comprise at least two domains: (1) a domain that shares homology to a target nucleic acid (e.g., and directs binding of a Cas9 complex to the target); and (2) a domain that binds a Cas9 (or similar) protein. In some embodiments, domain (2) is the sgRNA Backbone. In some embodiments, domain (2) corresponds to a sequence known as a tracrRNA, and comprises a stem-loop structure. For example, in some embodiments, domain (2) is homologous to a tracrRNA. In some embodiments, a gRNA comprises two or more of domains (1) and (2), and may be referred to as an "extended gRNA." For example, an extended gRNA will bind two or more Cas9 proteins and bind a target nucleic acid at two or more distinct regions. The gRNA comprises a nucleotide sequence that complements a target site, which mediates binding of the nuclease/RNA complex to said target site and providing the sequence specificity of the nuclease:RNA complex. The sequence of a gRNA that binds a target nucleic acid can comprise any sequence that complements a region of the target and is suitable for a nuclease:RNA complex to bind. In some embodiments, the RNA-programmable nuclease is the (CRISPR-associated system) Cas9 endonuclease, for example, Cas9 (Csnl) from Streptococcus pyogenes (see, e.g., "Complete genome sequence of an MI strain of Streptococcus pyogenes." Ferretti J. J., McShan W.M., Ajdic D.J., Savic D.J., Savic G., Lyon K., Primeaux C, Sezate S., Suvorov A.N., Kenton S., Lai H.S., Lin S.P., Qian Y., Jia H.G., Najar F.Z., Ren Q., Zhu H., Song L. expand/collapse author list McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma CM., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012)).

Because RNA-programmable nucleases (e.g., Cas9) use RNA:DNA hybridization to determine target DNA cleavage sites, these proteins are able to cleave, in principle, any sequence specified by the guide RNA. Methods of using RNA-programmable nucleases, such as Cas9, for site-specific cleavage (e.g., to modify a genome) are known in the art (see e.g., Cong, L. et al. Multiplex genome engineering using CRISPR/Cas systems. Science 339, 819-823 (2013); Mali, P. et al. RNA-guided human genome engineering via Cas9. Science 339, 823-826 (2013); Hwang, W.Y. et al. Efficient genome editing in zebrafish using a CRISPR-Cas system. Nature biotechnology 31, 227-229 (2013); Jinek, M. et al. RNA-programmed genome editing in human cells. eLife 2, e00471 (2013); Dicarlo, J.E. et al. Genome engineering in Saccharomyces cerevisiae using CRISPR-Cas systems. Nucleic acids research (2013); Jiang, W. et al. RNA-guided editing of bacterial genomes using CRISPR- Cas systems. Nature biotechnology 31, 233-239 (2013)).

The genetic construct according to the invention may comprise a gRNA expression cassette under control of an RNA promoter, preferably wherein the RNA promoter is a 7SK or U6 promoter. In other words in some embodiments the genetic construct of the invention may further comprise an RNA promoter operatively linked to the gRNA-expression construct. The person of skill knows various different promoters which could generally be used and depending on the application of the genetic construct may be varied accordingly.

By "RNA promoter" or "RNA pol III promoter" is meant any invertebrate, vertebrate, or mammalian promoter, e.g., human, murine, porcine, bovine, primate, simian, etc. that, in its native context in a cell, associates or interacts with RNA polymerase III to transcribe its operably linked gene, or any variant thereof, natural or engineered, that will interact in a selected host cell with an RNA polymerase III to transcribe an operably linked nucleic acid sequence. Included are U6 promoter (e.g., human U6, murine U6), H1 promoter, or 7SK promoter, which are meant to denote any invertebrate, vertebrate, or mammalian promoter or polymorphic variant or mutant found in nature to interact with RNA polymerase III to transcribe its cognate RNA product, i.e., U6 RNA, H1 RNA, or 7SK RNA, respectively.

In order to use the genetic construct in a genome editing application it is usually necessary that the editing nuclease is targeted to the nucleus of the target cell in order to access the target cell's genome. Therefore, the expressible sequence encoding for a gene editing nuclease in accordance with the herein described invention may comprise at least one nucleus localization signal (NLS) sequence, preferably wherein the at least one NLS sequence flanks the sequence encoding the nuclease 5' and/or 3' in frame. Several nuclear localization sequences (NLS) are known, and any suitable NLS can be used. For example, many NLSs have a plurality of basic amino acids, referred to as a bipartite basic repeats (reviewed in Garcia-Bustos et al, 1991, Biochim. Biophys. Acta, 1071 :83-101). An NLS containing bipartite basic repeats can be placed in any portion of the protein of the invention (the nuclease for example, but could also be used for other expressed proteins) and results in the protein being localized inside the nucleus. In preferred embodiments a nuclear localization domain is incorporated into the nuclease, as the ultimate functions of the nuclease described herein will typically require the proteins to be localized in the nucleus. However, it may not be necessary to add a separate nuclear localization domain in cases where expressible sequence itself, or another functional domain within the expressible sequence, has intrinsic nuclear translocation function, or where the amount of protein that passively reaches the nucleus is sufficient to perform the required function.

Further embodiments of the invention then pertain to the use of one or more additional selection marker to be comprised in the genetic construct of the invention, wherein the one or more additional selection marker is an expressible sequence encoding for a dominant negative mutant death receptor, wherein the parent death receptor is not identical to the parent death receptor of the selection marker. Preferably, the one or more additional selection marker and the selection marker are contained within one single ORF and separated in frame by a sequence encoding for a protein separation site as described herein before.

The genetic construct according to the invention may comprise a vector backbone, such as a viral vector backbone suitable for transfection of the target cell. The genetic construct shall not be limited to comprise a specific vector backbone, the skilled in the art is aware of various vectors that are usable for the transfection, either transient or stable.

The problem of the invention is furthermore solved by a recombinant cell comprising the genetic construct as described herein before. The term "recombinant cell line", refers to a cell line into which a recombinant expression vector such as the genetic construct of the invention has been introduced. It should be understood that "recombinant cell line" means not only the particular subject cell line but also the progeny of such a cell line. Because certain modifications may occur in succeeding generations due to either mutation or environmental influences, such progeny may not, in fact, be identical to the parent cell, but are still included within the scope of the term "recombinant cell line" as used herein. In some embodiments the recombinant cell is a prokaryotic cell used to propagate the genetic construct of the invention. Any suitable prokaryotic host cell may be used. In other embodiments the recombinant cell is a eukaryotic, preferably an animal cell. Such a cell could be identical to the target cell of the invention. In preferred embodiments the recombinant cell of the invention is a mammalian cell, such as a mouse, rat or human cell or cell line.

The object of the present invention is also solved by a transfection system, comprising a genetic construct as described herein before and a death receptor ligand, wherein the death receptor ligand is capable of binding the dominant negative mutant death receptor. In this aspect the transfection system is generally used for transfecting or transducing a cell with a genetic construct of the invention which could be modified to carry a nucleic acid sequence that is intended to be introduced into the target cell. The transfection system of the invention therefore provides the genetic construct as described above and which is used as vector for the nucleic acid sequence to be introduced into the target cells and which comprises the dominant negative mutant death receptor as a selection marker. Therefore, in order to allow the transfection system to be effective, the second component is a death receptor ligand which is capable of binding to and activating the parent death receptor from which the dominant negative mutant death receptor is derived. The transfection system is then used to transfect or transduct a target cell with the genetic construct of the invention (carrying a sequence as cargo) according to standard transfection or transduction protocols in the art. Most importantly the target cell carries a wild type version of the death receptor which can be activated by the death receptor ligand of the transfection system. Activation in this context means the induction of programmed cell death (apoptosis) via the respective death receptor. After the transfection procedure is completed the death receptor ligand of the transfection system is contacted with the transfected cells. In this way only such cells will escape the apoptosis activation that were positively transfected with the genetic construct of the invention because the dominant negative mutant death receptor expressed via the genetic construct as selection marker impairs death receptor signaling only in the transfected cell population. By culturing the transfected cells in the presence of the death receptor ligand, the percentage of positive transfectants can be increased.

In context of the present invention the inventors intended to provide additional tools for potent and fast enrichment of gene-edited cells, and thus implemented the present original strategy based on killing of transfected cells that lack Cas9 expression through the extrinsic apoptosis signaling pathway. The inventors hypothesized that co-expression of the Cas9 nuclease with such receptor mutants would render cells resistant to CD95L- or TRAIL-induced apoptosis, and that incubation with the appropriate ligand would allow for the enrichment of Cas9-positive cells. In vitro, killing cells can be efficiently achieved by using modified forms of soluble CD95 and TRAIL ligands that are, for example, fused to an isoleucine zipper. Ligands can be produced in-house in a straightforward and inexpensive manner, do not require purification and kill cells within a few hours. A proof-of-concept is provided in the example section. Although the basic concept of the invention was firstly realized in context of a genome editing application by enriching CAS9 transfected cells, the general idea of using the dominant negative mutant death receptor as a selection marker could be transferred to many other applications where a nucleic acid sequence is introduced into a target cell and wherein an enrichment of positively transfected cells is beneficial.

In preferred embodiments of the invention the transfection system comprises as death receptor ligands a soluble death receptor ligand (which are known in the art). More preferred is a molecule selected from CD95 ligand (CD95L) or TNF-related apoptosis-inducing ligand (TRAIL), or soluble variants or orthologs of these molecules.

In preferred embodiments of the invention the death receptor comprised in the genetic construct is DR4 or DR5, and the death receptor ligand is TRAIL or soluble TRAIL (sTRAIL). Alternatively the death receptor comprised in the genetic construct is CD95 and the death receptor ligand is CD95L or soluble CD95L (sCD95L), or orthologs/homologs of these molecules. In order to allow for an excellent activation of the death receptor, respectively its inhibition by the dominant negative mutant, the death receptor ligand and the dominant negative mutant death receptor are derived from the same species.

The transfection system described herein is preferably for use in the transfection of a target cell, the target cell comprising an endogenously expressed wild-type death receptor that corresponds to the dominant negative mutant death receptor (or is the "parent" molecule of the dominant negative mutant death receptor).

Another aspect of the invention then relates to a method for introducing a genetic sequence into a target cell, the method comprising the steps of

(a) Introducing a genetic sequence of choice into the genetic construct according to the invention,
(b) Transfecting or transducing a culture of the target cell with the genetic construct obtained from (a),
(c) Culturing the transfected or transduced target cells of (b) in the presence of a death receptor ligand for a time period sufficient to induce apoptosis in non-transfected or non-transduced cells,
(d) Retrieving the living target cells from (c).

The introduction of the genetic sequence of choice into the genetic construct of the invention is preferably done by molecular cloning techniques which are standard procedures for the skilled artisan.

The transfecting or transducing may be performed either transiently or stably. However, transient transfection is preferred in the context of genome editing in order to avoid unnecessary modifications of the genome of the target cells. However, also stable integrations into the genome of the target cells could be used. Then, the genetic construct, comprising the genetic sequence to be introduced, is for example transduced into the target cells by lentiviral transduction in step (b).

One of the advantages of the dominant negative mutant death receptor as selection marker in accordance with the invention is the surprisingly fast selection process. Therefore, the time period for culturing the transfected or transduced target cells of step (b) in the presence of a death receptor ligand sufficient to induce apoptosis in non-transfected or non-transduced cells is preferably less than 30 hours, preferably less than 20 hours, more preferably less than 10 hours, most preferably is about 5 hours.

In some preferred embodiments the target cell endogenously expresses the wild type complement to the dominant negative mutant death receptor comprised in the genetic construct. Preferably the death receptor ligand binds to the dominant negative mutant death receptor and its wild type complement, and wherein the presence of the dominant negative mutant death receptor in the cell inhibits the activation of the wild type death receptor.

The death receptor or death receptor ligand are selected as described herein before.

The problem is furthermore solved by a method for CRISPR mediated editing of the genome of a target cell, comprising transfecting/transducing a genetic construct according to the invention into the target cell to be edited, contacting the transfected/transduced hosts cell with a death receptor ligand for a time period sufficient to induce apoptosis in the target cell, retrieving a culture of target cells enriched for positively transfected/transduced target cells. As described before, by contacting the transfected cells with the death receptor ligand apoptosis is induced and only cells transfected with the genetic construct of the invention are able to escape the death receptor ligand mediated signal, and therefore escape cell death. In consequence, transfected cells are enriched upon contacting with the death receptor ligand.

A preferred embodiment of the method pertains to the concomitant use of a compound that is capable of sensitizing cells to death receptors, such as cycloheximide or similar compounds. This concomitant use is applicable to all aspects and embodiments of the invention.

Yet another aspect of the invention pertains to a method for selecting transfected/transduced target cells, the method comprising

(a) Transfecting/transducing the target cell with a genetic construct according to the invention, and
(b) Contacting the target cell with a death receptor ligand for a time period sufficient to induce apoptosis in the target cell.

Also this method may include a further step of sensitizing target cells by contacting the target cells with a compound that is capable of sensitizing cells to death receptors, such as cycloheximide or similar compounds.

Therefore in another aspect, the invention relates to the use of the genetic construct or transfection system of the invention described before in genome editing, preferably in CRISPR/Cas9 mediated genome editing.

The present invention will now be further described in the following examples with reference to the accompanying figures and sequences, nevertheless, without being limited thereto. For the purposes of the present invention, all references as cited herein are incorporated by reference in their entireties.

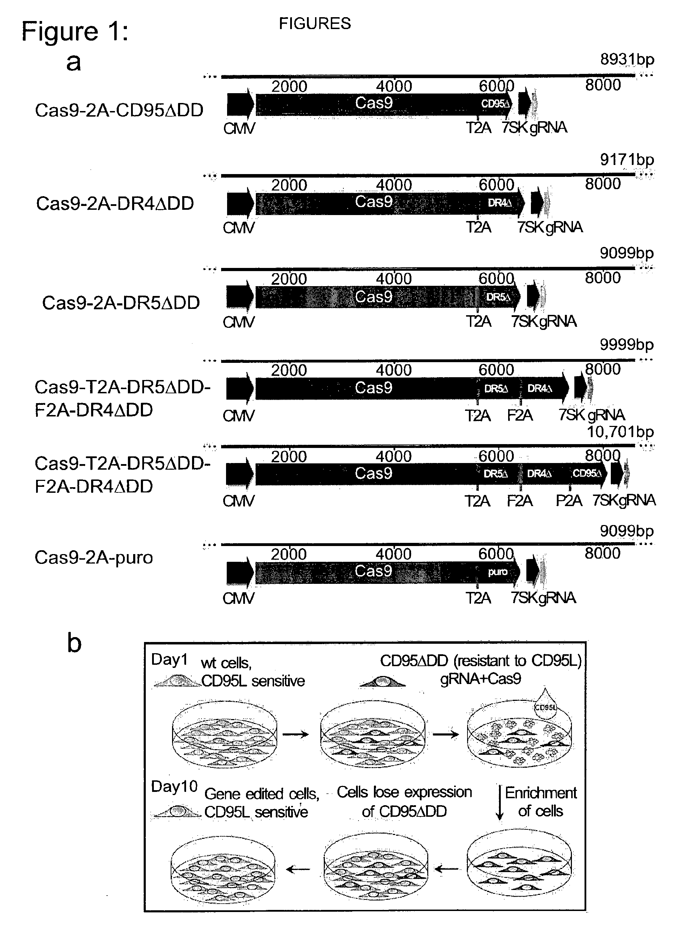

Figure 1: Constructs and workflow of death receptor-based enrichment of Cas9-expressing cells. (a) Constructs for different selection strategies. The Cas9 nuclease is linked to the resistance gene by a cleavable 2A peptide allowing stoichiometric expression. As resistance gene, the inventor used death receptors that lack the intracellular death domain, denoted by Δ. CD95 is chosen when using CD95L as selection agent. DR4 and DR5 are death receptors that bind the death ligand TRAIL. To allow simultaneous expression of several death receptors, the inventors linked them to Cas9 via the 2A peptide from Thosea asigna virus (T2A), from foot-and-mouth disease virus (F2A) and porcine teschovirus-1 (P2A). To allow comparison to the puromycin selection strategy, the inventors cloned the puromycin resistance gene puromycin N-acetyl-transferase, denoted here as puro. The inventors used the human cytomegalovirus (CMV) and the 7SK promoters for the protein and gRNA expression, respectively. (b) Workflow. Cells are transfected with a plasmid encoding the Cas9 nuclease, a selection gene and the gRNA. Two days after transfection, death ligand was added to the cells, before they were washed and expanded.

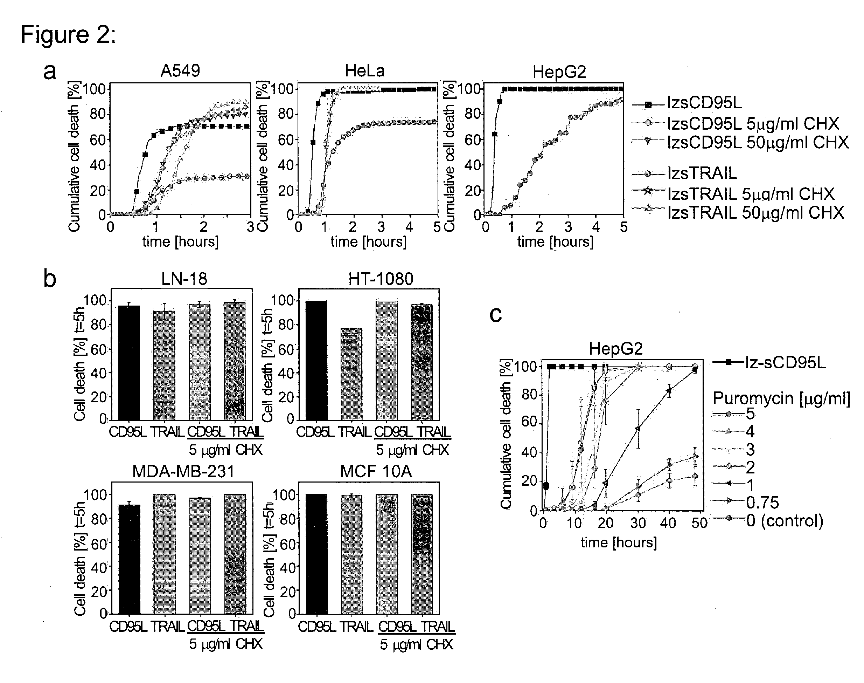

Figure 2: Death ligands induce cell death in various cell types within a few hours. Cell death was measured by observing cell morphology by microscopy. (a) Cell death kinetics of A549, HepG2 and HeLa cells using IZsTRAIL and IZsCD95L, with and without co-treatment with 5 to 50 μg/ml cycloheximide (CHX). (b) Cell death of LN-18, HT-1080, MDA-MB-231 and MCF10A cells treated for 5 h with IZsCD95L (denoted CD95L) or IZsTRAIL (denoted TRAIL), with or without 5 μg/ml CHX. (c) HeLa cell death kinetics comparison between IZsCD95L and puromycin. While cell death occurred after 2 hours with IZsCD95L, it saturated at 30 hours with puromycin. b and c: mean ± s.d. of 3 different fields of view with at least 50 cells each.

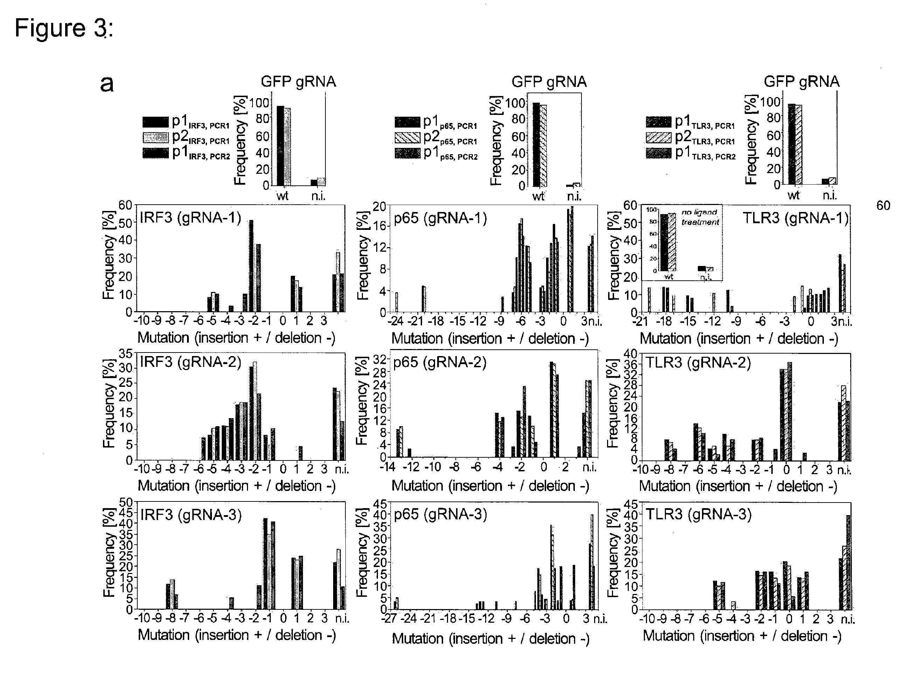

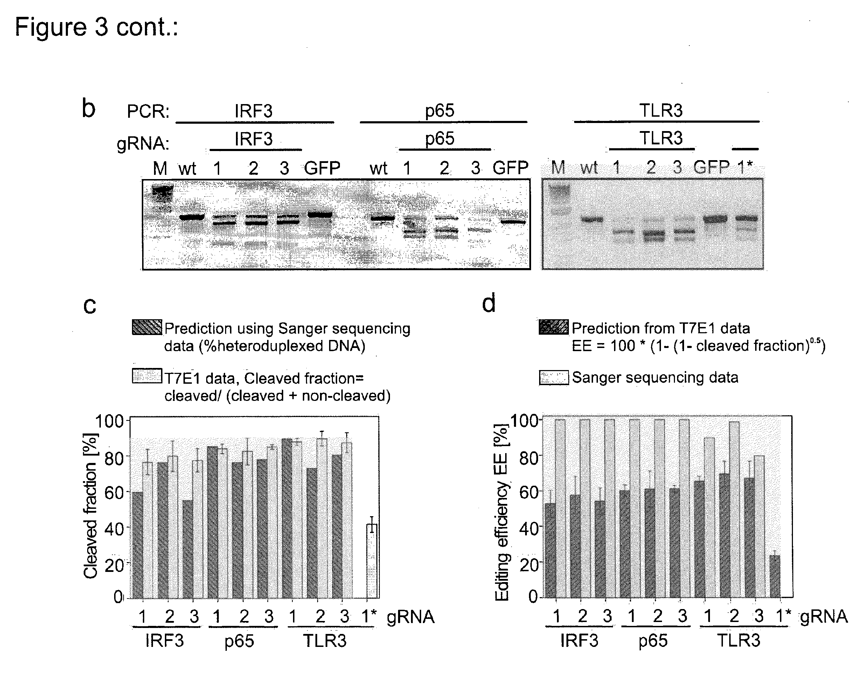

Figure 3: Editing of IRF3, p65 and TLR3 genes in HeLa cells using the Cas9-T2A-CD95Δ construct and IZsCD95L as selection agent. Three different gRNAs per gene were tested and a control gRNA targeting GFP was used. Cells denoted as TLR3 gRNA-1* were not treated with IZsCD95L. (a) Sanger sequencing results. The frequency of indels in polyclonal cell lines was quantified from chromatograms using the TIDE analysis. Genome extraction, PCR and sequencing were performed twice. PCR1 was in addition sequenced with a second primer (p2). Mutation = 0 represents wild type sequence. The non-interpretable fraction (n.i.) relates to the correlation coefficient of the TIDE analysis with R2 = 100 - n.i. (b) Representative agarose gels from three T7E1 assays. Genome extraction from polyclonal HeLa cell lines, PCR and T7E1 digest were repeated three times. (c) Predictions of the cleavage fraction were obtained by estimating the amount of heteroduplexed DNA by using equation (2) and data from the Sanger sequencing (green-striped). The cleavage fraction (yellow bars) was quantified from T7E1 assays by: cleaved DNA/(cleaved DNA+non-cleaved DNA). (d) Purple-striped bars show the predicted editing efficiency using equation (1) and data from the T7E1 assay. A different estimate of the editing efficiency was obtained by Sanger sequencing and the TIDE analysis (grey bars).

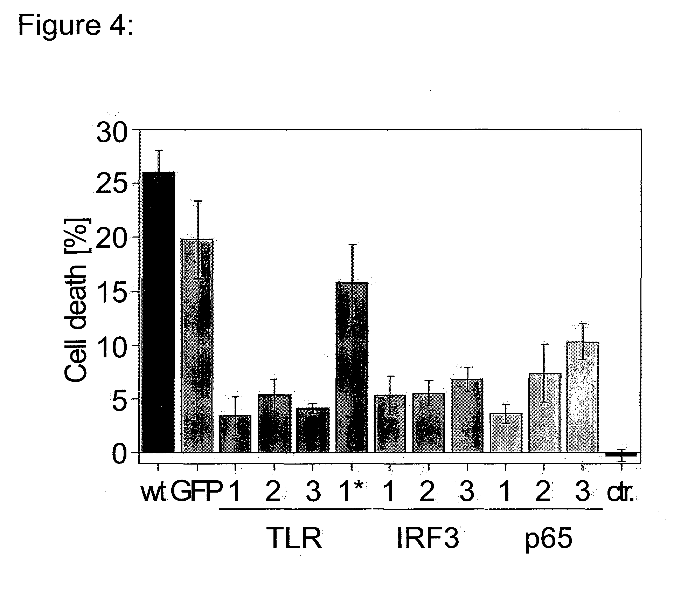

Figure 4: Poly (I:C)-induced cell death is reduced in cells with impaired TLR3, IRF3 and p65 expression. 24 h after transfection of 2 μg/ml Poly (I:C) with DOTAP, fractional cell death was measured by flow cytometry using propidium iodide. The inventors compared wt Hela cells and polyclonal HeLa cell lines generated with the nine different gRNAs targeting TLR3, IRF3 and p65 genes and the control gRNA targeting GFP. TLR3 gRNA-1 cells denoted with a star (*) were not treated with IZsCD95L, while ctr. denotes HeLa wt cells transfected with a non-triphosphate 19-mer RNA control to account for cell death due to transfection with DOTAP. Shown are means ± s.e.m. of 3 independent experiments.

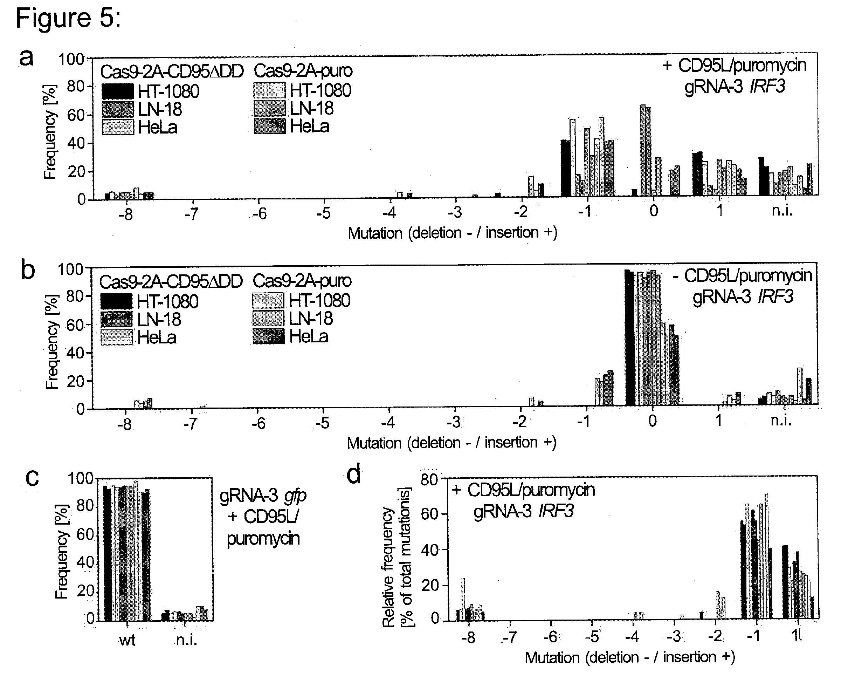

Figure 5: Death receptor-based and puromycin-based enrichment of Cas9-expressing cells. In two independent experiments, HT-1080, LN-18 and HeLa cells were transfected with plasmid Cas9-2A-CD95ΔDD or Cas9-2A-puro encoding gRNA-3 targeting the IRF3 gene or gRNA targeting gfp. Cell lines were tested against wild type cells for presence of indels in the gene IRF3 using primer IRF3 p1 (Table 1). Indels were detected by Sanger sequencing and TIDE analysis. (a) Selection using IZsCD95L or 5 μg/ml puromycin treatment. (b) No selection. (c) gfp gRNA cells treated with IZsCD95L or 5 μg/ml puromycin. Mutation = 0 represents wild type sequence. The non-interpretable fraction (n.i.) relates to the correlation coefficient with R2 = 100 - n.i. (d) The relative frequency of indels in enriched cells was calculated by dividing their amplitude from panel 5a by the sum of all indels.

EXAMPLES

**Materials and Methods**

Cell culture

MDA-MB-231 and HT1080 cells were obtained from CLS (Eppelheim, Germany). LN-18 cells were a kind gift from Ana Martin-Villalba. MCF-10A cells were obtained from ATCC (Manassas, VA, USA). HeLa cells were the so-called HeLa Kyoto and were obtained from Holger Erfle. A549 and 293T cells were kind gifts from Ralf Bartenschlager and Dirk Grimm, respectively. HeLa, HT-1080, MDA-MB-231, LN-18, 293T and A549 cells were maintained in Dulbecco's modified eagle medium (DMEM, Life Technologies, Darmstadt, Germany) containing 10% fetal calf serum (Biochrom AG, Berlin, Germany), penicillin/streptomycin (100 μg/ml each, Life Technologies). MCF 10A cells were maintained in DMEM with F12 (Life Technologies), 5% horse serum (Biochrom AG), 20 ng/ml EGF (TEBU-Bio, Offenbach, Germany), 0.5 μg/ml hydrocortisone (Sigma-Aldrich, St. Louis, Missouri, USA), 10 μg/ml insulin (Sigma-Aldrich) and penicillin/streptomycin (100 μg/ml each, Invitrogen). For ligand production, 293T cells were transfected using branched polyethylenimine (PEIpro, PolyPlus, Illkirch, France). X-tremeGENE was used for the transfection of the Cas9 constructs, while Polyinosinic:polycytidylic acid (Poly(I:C)) and 5'ppp-dsRNA control (Invivogen, San Diego, CA, USA) were transfected with DOTAP (Carl Roth, Karlsruhe, Germany). Puromycin was from Life Technologies.

Plasmids

DR4-ΔDD and DR5-ΔDD were amplified from Gateway full ORF clones from the genomics and proteomics core facilities of the DKFZ, Heidelberg. CD95-ΔDD was amplified from the full-length coding sequence of CD95. DR4-ΔDD, DR5-ΔDD and CD95-ΔDD contain the first 290, 250 and 210 amino acids of the full-length human DR4, DR5 and CD95, respectively. The gene coding for puromycin N-acetyl-transferase was amplified from the Cas9-2a-puro plasmid used in Shalem et al. Science 2014, 343(6166):84-87. (originally Addgene #49535). The Cas9 sequence is the one derived from Streptococcus pyogenes originally used in Shalem et al. 2014 (Addgene #49535). It is preceded by a CMV promoter, and followed by a SV40 nuclear localization sequence, the 2A peptide from Thosea asigna virus T2A EGRGSLLTCGDVEENPGP (SEQ ID NO: 1), a peptide linker RSMH and the cDNA of one of the four resistance genes without the first methionine-encoding triplet. In the Cas9-T2A-DR5ΔDD-F2A-DR4ΔDD construct, the DR5ΔDD and DR4ΔDD are separated by the foot-and-mouth disease virus 2A peptide F2A VKQTLNFDLLKLAGDVESNPGP (SEQ ID NO: 2) preceded by the linker SG and followed by the linker RSMH. In construct Cas9-T2A-DR5ΔDD-F2A-DR4ΔDD-P2A-CD95ΔDD, the additional CD95ΔDD is separated from DR4ΔDD by the porcine teschovirus-1 2A peptide P2A ATNFSLLKQAGDVEENPGP (SEQ ID NO: 3), and preceded by the linker GSG and followed by the linker RSMH. The coding sequence for the guide RNA was cloned on the same plasmid and driven by a 7SK promoter. gRNA design was performed using the ZiFiT Targeter software. All constructs were cloned based on the pSSV9 vector (Samulski RJ, Chang LS, Shenk T: A recombinant plasmid from which an infectious adeno-associated virus genome can be excised in vitro and its use to study viral replication. J Virol 1987, 61(10):3096-3101), in which the entire cassette consisting of the Cas9 and gRNA expression units was inserted between the two XbaI sites.

IZsCD95L (Walczak H, Miller RE, Ariail K, Gliniak B, Griffith TS, Kubin M, Chin W, Jones J, Woodward A, Le T et al: Tumoricidal activity of tumor necrosis factor-related apoptosis-inducing ligand in vivo. Nature Medicine 1999, 5(2):157-163), was cloned into pIRES-puro2 (Clontech Laboratories, Inc., CA, USA), and IZsTRAIL was built from IZsCD95L in pIRES-puro2. In the following the amino acid sequence for IZsCD95L and IZsTRAIL in one-letter code starting from the N-terminus is given. To allow protein secretion, the inventors used the N-terminal signal sequence MGTPHLQGFLLLLFPLLLRLHGASAGS (SEQ ID NO: 4) in the construct IZsTRAIL, and the signal sequence MARRLWILSLLAVTLTVALAALE (SEQ ID NO: 5) in the construct IZsCD95L. In both constructs, those were followed by the Flag tag DYKDDDDK (SEQ ID NO: 6) and amino acids PSQKSKRRTSSDRMKQIEDKIEEILSKIYHIENEIARIKKLIGERTR (SEQ ID NO: 7) encoding the isoleucine-zipper domain (PDB entry 1GCM). In IZsCD95L, the sequence encoding CD95L from amino acids 117-281 (STSQ...LYKL) was used. In IZsTRAIL, the linker SGGSSG bridges the IZ domain to the extracellular receptor binding domain of TRAIL from amino acids 122-281 (VAAH...FLVG). The sequence of TRAIL was amplified by PCR from cDNA of NK92-C1 cells.

Western blot

Western blots were performed by lysing cells using ice-cooled lysis buffer (20 mM Tris/HCl, pH 7.5, 150 mM NaCl, 1 mM phenylmethylsulfonyl fluoride (Sigma-Aldrich), protease inhibitor cocktail, 1% Triton X-100 (Serva, Mannheim, Germany), and 10% glycerol). Lysates were analyzed using SDS-PAGE gels (Novex NuPAGE 10% Bis-Tris Protein Gel, Life Technologies). Proteins were transferred to PVDF membrane (Millipore, Billerica, MA, USA) using wet blotting. IRF3 was detected using the primary antibody clone D83B9 (Cell Signaling Technology, Danvers, MA, USA). Secondary anti-rabbit antibodies were HRP-conjugated (Dianova, Hamburg, Germany). Detection was performed using the Pico Chemiluminescent Substrate from Thermo Scientific (Asheville, NC, USA) and a CCD camera.

Immunofluorescence

CD95, DR4 and DR5 were detected in living cells using the antibody clones DX2, DJR1 and DJR2-4, respectively, each labeled with phycoerythrin (PE) (eBioscience Inc, San Diego, CA, USA). Cells were trypsinized, blocked on ice with 1% bovine serum albumin (BSA, Sigma) in PBS, incubated for 30 minutes with antibody in blocking buffer on ice and directly measured by flow cytometry (Beckman Coulter, Krefeld, Germany).

Poly (I:C) treatment

75000 cells were seeded into wells of a 24-well plate and treated one day later by transfection of 2 $\mu$g/ml (final concentration, f.c.) Poly (I:C). 24 hours later, dead and living cells were collected. Cell supernatant, potentially containing living and dead cells, was transferred into empty wells of a 24-well plate. Remaining cells were washed once with PBS and trypsinized. PBS and trypsinized cells were merged with the previously collected cell supernatant. Sample was incubated with propidium iodide (solution, 1 $\mu$g/ml f.c., Sigma-Aldrich) 10 minutes before measurement by flow cytometry (Beckman Coulter).

Editing analysis

Genomic DNA was extracted using the DirectPCR® Lysis-Regent Cell (Peqlab, Erlangen, Germany) according to the protocol. PCRs were performed using the Phusion Flash PCR master mix (Thermo Scientific). T7 endonuclease I was from NEB (Ipswich, MA, USA). Oligonucleotides were from Eurofins MWG Operon (Ebersberg, Germany), and Sanger sequencing was performed by GATC Biotech (Konstanz, Germany).

Microscopy

Cell death was measured by microscopy using transmission imaging and visual cell morphology identification. Images were taken on a Leica SP5 confocal microscope (Leica Microsystems, Mannheim, Germany) or on an Olympus CKX41 wide-field microscope equipped with an Olympus PEN Lite CCD camera (Olympus Europa, Hamburg, Germany).

Computer simulations

Simulations to predict the T7E1 cleavage fraction from indel patterns were performed using MATLAB (The MathWorks GmbH, Ismaning, Germany).

**Example 1: Death receptor-based selection of Cas9-expressing cells**

The inventors established a death receptor-based selection system that enriches Cas9-expressing cells in order to generate polyclonal, gene-edited cell populations. To this end, a single plasmid encoding gRNA and Cas9 nuclease together with the selection gene that is co-expressed via a 2A peptide was designed, adapted from Shalem et al. Science 2014 (Figure 1a). Using death ligands as selection agent, the inventors aimed at killing non-transfected cells and cells expressing too low levels of Cas9 nuclease. As selection genes, truncations of death receptors were used that are unable to transmit the apoptotic signal (see workflow on Figure 1b). More precisely, the inventors made truncated receptors missing their death domain (ADD) for the death ligand CD95 ligand (CD95-ADD) or TRAIL (DR4-ADD and DR5-ADD) (Figure 1a). Their single expression as well as the simultaneous expression of both, DR4-ADD and DR5-ADD, or even of all three mutant receptors, CD95-ADD, DR4-ADD and DR5-ADD, from the same construct was tested. As detected by immunofluorescence, all vectors robustly over-expressed the mutant receptors in the different cell lines that were tested, HeLa, HT-1080, LN-18 and MDA-MB-231.

Since the object of the present invention was to develop a strategy that would minimize the requirements for production of reagents, the inventors next tested the possibility to directly use secreted, non-purified ligands expressed in HEK 293T cells. The apoptotic activity of soluble death ligands (sCD95L and sTRAIL) that are fused to the isoleucine-zippper domain (IZ), denoted as IZsCD95L and IZsTRAIL, was measured in different human cell lines: HeLa, HT-1080, LN-18, MDA-MB-231, HepG2, MCF10A and A549 (Figure 2a and b). With the exception of A549, all cell lines showed more than 99% cell death within 5 hours with at least one of the ligands. Remarkably, 1 hour was even sufficient to kill HeLa and HepG2 cells. Addition of 5 or 50 $\mu$g/$\mu$l cycloheximide, known to amplify the sensitivity to death receptors, could further enhance cell death in some cases. Still, in all cells except A549, one of the two ligands efficiently induced cell death without cycloheximide (Figure 2), showing that those ligands can be directly used as strong selection agent. Notably, cell death induced by puromycin, even at saturating concentrations, required about 30 hours to kill more than 99% HeLa, LN-18 and MDA-MB-231 cells (Figure 2c). Therefore, cell death can be more efficiently induced by death ligands than by puromycin.

**Example 2: Estimating genome editing efficiency in HeLa cells**

In order to test the capacity of the inventive death receptor strategy to efficiently generate gene-edited cells, three different genes in HeLa cells, IRF3, TLR3 and p65, were targeted with three different gRNAs for each gene. As CD95L killed HeLa cells more efficiently than TRAIL (Figure 2a), the Cas9-2a-CD95ΔDD construct was used. As mock control, the inventors used a gRNA that was designed to target gfp (see Table 1 for the primer and gRNA sequences used for this invention). Two days after transfection, cells were incubated for 5 hours with IZsCD95L. Cells were also kept transfected with TLR3 gRNA-1 untreated, denoted as TLR3 gRNA-1*. Seven days after treatment, genomic DNA was extracted for analysis and the remaining cells were kept in culture for direct phenotyping. The editing efficiency was evaluated, denoting the fraction of mutant DNA species, by using two different methods, namely the analysis of Sanger sequence chromatograms (Figure 3a) and the T7E1 assay (Figure 3b). To quantify the mutations from sequencing chromatograms, the inventors applied the TIDE (Tracking of Indels by DEcomposition) analysis, a sequence decomposition approach. To this end, the genomic region targeted by the different gRNAs in the polyclonal HeLa cell lines was PCR-amplified. The three gRNAs for one gene were located in the same region of the genome, hence the same primers for each gene were used. To check the consistency of the indel calculation, each PCR product was sequenced from both sides of the cut (Table 1). In all cases, sequencing chromatograms already provided a clear visual impression of the presence of genetic modifications, mostly evidenced by a unique sequence before the cutting site and a mixture of sequences behind it. In some cases, a small amount of mutated sequences was also detected before this cutting site, which likely corresponds to large indels that start after the sequencing primer. Strikingly, in cell lines enriched for IRF3 and p65 cleavage, no wt sequence of the respective genes was detected (Figure 3a), while the amount of wt TLR3 sequence was 8 to 36%. In contrast, no indels were identified in non-enriched TLR3 gRNA-1* cells or in enriched gfp gRNA control cells (Figure 3a, TLR3 gRNA-1 inset and upper plots). Therefore, this first approach indicated efficient enrichment of gene-edited cells.

Using the T7E1 assay, fractions of cleaved PCR product was obtained (cleaved/[cleaved+noncleaved]) ranging between 77% and 90% (Figure 3b and c, yellow bars). In contrast to the sequencing results, cells that were not treated with IZsCD95L still showed 41% cleavage. The editing efficiency is typically calculated from the fraction of cleaved PCR products as follows

$$\text{editing efficiency} = 1 - \sqrt{1 - fraction_{cleaved}} \qquad \text{Equation (1)}$$

Following this estimation, editing efficiencies ranging between 53% and 70% for cells treated with IZsCD95L and 23% for non-selected cells was obtained (Figure 3d). Thus, both approaches verified successful editing and enrichment of cells. Yet, they showed a large discrepancy in the editing efficiency for enriched cells, reaching up to 100% for the sequencing approach versus up to 70% for the T7E1 approach. In contrast, non-enriched cells showed 0% editing by sequencing but 23% by the T7E1 approach (Figure 3d, TLR3 gRNA-1*).

These discrepancies led the inventors to revisit the estimation of the editing efficiency. On the one hand, the Sanger sequencing might not be sensitive enough to detect a low fraction of mutant DNA species in the non-enriched sample, and vice versa, to detect a low amount of wt sequences in the enriched samples. On the other hand, the editing efficiency calculated using equation (1) relies on the assumption that the diversity of mutated strands is sufficiently large so that those mutated strands never reanneal with strands carrying the same mutation. In other terms, only the wt strands would form homoduplexes and would remain uncleaved by the endonuclease. As a low diversity of indels by sequencing was identified, this questioned the validity of the aforementioned assumption. In order to test it, the inventors calculated the expected cleavage fraction in an endonuclease assay by assuming that not only wt but also mutant homoduplexes can form. This can be expressed as follows:

$$fraction_{cleaved} \approx fraction_{heteroduplex} = 1 - \sum_{i=1}^{m} p_j^2 \quad \text{Equation (2)}$$

where m is the number of possible sequences, with m-1 mutants and one wt, and pj is the relative amount of each of the sequences. This calculation shows that the cleavage fraction would not reach 100% even when no wt sequence is present. It also shows that for an editing efficiency below 30%, the original equation (1) is particularly while for high editing efficiencies it becomes unsuitable.

To test the relevance of this calculation in practice, the inventors hypothesized that the measured frequency of indels by the TIDE analysis mirrors the likelihood to obtain a mutation for a particular gRNA. Therefore, equation (2) was applied using the frequencies shown in Figure 3a to derive the expected cleavage fraction in a T7E1 assay. Strikingly, the resulting predictions (Figure 3c, green striped bars) were in good agreement with the measured cleavage fractions (Figure 3c, yellow bars). Also the endonuclease-mediated cleavage depending on varying amount of wt sequence was estimated. By this means, the inventors observed that the cleavage fraction cannot exceed 70% to 90% depending on the gRNA. Thus, the sensitivity of the Sanger sequencing and the aforementioned limitations of the interpretation of the T7E1 assay might readily explain the discrepancies in the estimation of the editing efficiency. Importantly, irrespective of the true editing efficiency, both approaches confirmed a strong enrichment of gene-edited cells when using CD95-induced apoptosis as selection strategy.

**Example 3: Phenotyping of knock-out effects after death receptor-based enrichment**

As a proof-of-concept, the different HeLa cell lines were used for a direct phenotyping of introduced gene mutations. Specifically, the inventors were interested in the role of p65, IRF3 and TLR3 in double-stranded (ds)RNA-induced apoptosis. Some previous studies reported the requirement of transcriptional activity through p65 and/or IRF3 for this type of death, while other reports showed the possibility of direct cell death through the formation of the ripoptosome on activated TLR3. Notably, this cell death response typically only occurred in a fraction of the cell population. To investigate the role of the three genes in dsRNA-induced death in HeLa cells, the death response of edited cells after Poly (I:C) treatment was quantified by flow cytometry. Using wt HeLa cells and gfp gRNA-treated cells as controls, a cell death of 26.0±2.0% and 19.8±3.6%, respectively was obtained. Interestingly, in p65, IRF3 and TLR3 gene-edited cell lines, the cell death response was strongly reduced, ranging between 3.4±1.8% with TLR3 gRNA-1 and 10.3±1.6% with p65 gRNA-3 (Figure 4). Notably, still 15.7±3.6% of non-enriched TLR3 gRNA-1* cells were killed by Poly (I:C) treatment. Together, this emphasizes that the three genes are involved in this type of death and that enrichment of gene-edited cells helps to directly assess the associated cellular phenotype.

**Example 4: Comparison of cell enrichment by death receptors versus Puromycin**

Finally, the gene editing efficiency in HeLa, HT-1080 and LN-18 cells when using the death receptor-based versus the puromycin-based selection approach was tested. For this, the gene IRF3 was targeted using gRNA-3 either with the Cas9-2a-CD95ΔDD plasmid used above, as CD95L was most potent to kill those cell lines, or with the Cas9-2a-puro plasmid (shown in Figure 1a). Two days after transfection with Cas9-2a-CD95ΔDD, cells were incubated with IZsCD95L for 5 hours or left untreated. HeLa, HT-1080 and LN-18 cells transfected with Cas9-2a-puro were treated for 30 hours with 5, 3 and 5 μg/ml puromycin, respectively, or left untreated. The concentrations were chosen to ensure a complete killing of the cells (Figure 2c). Cells were then maintained in culture until the density of all treated cells allowed their characterization, between 7 and 11 days post-transfection.

As assessed by the TIDE analysis of sequenced PCR products, different efficiencies depending on the cell type and the selection strategy were obtained (Figure 5). For each condition, the fraction of indels was calculated by dividing their amount by the amount of wt and indel sequences (Figure 5a). For death receptor-enriched cells, the fraction of indels in HT-1080 cells was 100% and 94%, in LN-18 cells 29% and 25%, and in HeLa cells 100% and 100%, respectively, in two biological replicates each. For puromycin-enriched cells, HT-1080 cells showed 100% indels, LN-18 cells 94% and 65%, and HeLa cells 80% and 73%. In contrast, in non-enriched transfected cells, indels were detected only in one replicate of LN-18 cells transfected with Cas9-2a-puro (2.2%), in HeLa cells with Cas9-2a-puro (41% and 39%), and in HeLa cells with Cas9-2a-CD95ΔDD (40% and 33%) (Figure 5b). As expected, enriched and transfected control cells (gfp gRNA) displayed only wt sequences (Figure 5c).

This invention demonstrates the usefulness of death receptors as tool for the selection of Cas9-expressing cells, and the use as a selection marker in general. Killing non-transfected cells with death ligands can trigger enrichment of gene-edited cells that allows for direct assessment of gene function. The here presented extension of the tool panel based on the CRISPR/Cas9 technology will particularly enhance low- and high-throughput phenotyping screening after CRISPR-mediated gene knockdown.

**Abbreviations**

AAV: Adeno-associated virus

CRISPR: clustered regularly interspaced short palindromic repeats

DD: death domain

DR: death receptor

gRNA: single guide RNA

indel: insertion/deletion

TIDE: Tracking of Indels by DEcomposition

T7E1: T7 endonuclease I

wt: wild type

# EP 3 147 366 A1

**Claims**

1. A genetic construct for transfection of a target cell, comprising an expression cassette and a selection marker, **characterized in that** the selection marker is an expressible sequence encoding for a dominant negative mutant death receptor.

2. A genetic construct according to claim 1, for clustered regularly interspaced short palindromic repeats (CRISPR) - mediated gene editing in a target cell, wherein the expression cassette comprises an expressible sequence encoding for a genome editing nuclease, preferably a genome editing nuclease selected from TALEN, ZNF-nucleases or CRISPR nucleases, or a functional variant thereof.

3. The genetic construct according to claim 2, wherein the genome editing nuclease is CRISPR associated protein 9 (CAS9), or a homolog or ortholog thereof, or is a deactivated Cas9 (dCas9), preferably a fused to a protein with an enzymatic activity other than a nuclease.

4. The genetic construct according to any of claims 1 to 3, wherein dominant negative mutant death receptor is a death receptor comprising a mutated and inactive death domain, preferably comprising a death domain deletion (ΔDD).

5. The genetic construct according to any of claims 1 to 4, wherein the death receptor is selected from Fas/CD95, DR4 or DR5.

6. The genetic construct of any of claims 2 to 5, wherein the expressible sequence encoding for a CRISPR nuclease and the expressible sequence encoding for a dominant negative mutant death receptor are contained within a single open reading frame (ORF) encoding for a fusion protein composed of the CRISPR nuclease and dominant negative mutant death receptor, and wherein the two expressible sequences within the single ORF are separated in frame by a sequence encoding for a protein separation site.

7. The genetic construct according to any of claims 2 to 6, further comprising a guide RNA (gRNA) - expression cassette, composed of an invariant gRNA scaffold and a cloning site for insertion of gRNA sequence.

8. A transfection system, comprising a genetic construct according to any of claims 1 to 7 and a death receptor ligand, wherein the death receptor ligand is capable of binding the dominant negative mutant death receptor.

9. The transfection system according to claim 8, wherein the death receptor ligand is selected from CD95 ligand (CD95L) or TNF-related apoptosis-inducing ligand (TRAIL), or soluble variants thereof.

10. The transfection system according to claim 8 or 9, wherein the death receptor comprised in the genetic construct is DR4 or DR5, and the death receptor ligand is TRAIL or soluble TRAIL (sTRAIL); or wherein the death receptor comprised in the genetic construct is CD95 and the death receptor ligand is CD95L or soluble CD95L (sCD95L), or orthologs of these molecules.

**11.** The transfection system according to any of claims 8 to 10, wherein the death receptor ligand and the dominant negative mutant death receptor are derived from the same species.

**12.** A method for introducing a genetic sequence into a target cell, the method comprising the steps of

(a) Introducing a genetic sequence of choice into the genetic construct according to any of claims 1 to 7,
(b) Transfecting or transducing a culture of the target cell with the genetic construct obtained from (a),
(c) Culturing the transfected or transduced target cells of (b) in the presence of a death receptor ligand for a time period sufficient to induce apoptosis in non-transfected or non-transduced cells,
(d) Retrieving the living target cells from (c).

**13.** The method according to claim 12, wherein the death receptor ligand binds to the dominant negative mutant death receptor and its wild type complement, and wherein the presence of the dominant negative mutant death receptor in the cell inhibits the activation of the wild type death receptor.

**14.** The method according to claim 12 or 13, wherein the death receptor is selected from CD95, DR4 or DR5, and the death receptor ligand is selected from CD95L or TRAIL, orthologs thereof, or soluble variants thereof.

**15.** A method for CRISPR mediated editing of the genome of a target cell, comprising transfecting/transducing a genetic construct according to any of claims 2 to 7 into the target cell to be edited, contacting the transfected/transduced hosts cell with a death receptor ligand for a time period sufficient to induce apoptosis in the target cell, retrieving a culture of target cells enriched for positively transfected/transduced target cells.

**16.** A method for selecting transfected/transduced target cells, the method comprising

(a) Transfecting/transducing the target cell with a genetic construct according to any of claims 1 to 7,
(b) Contacting the target cell with a death receptor ligand for a time period sufficient to induce apoptosis in the target cell.

FIGURES

# Figure 1:

Figure 2:

EP 3 147 366 A1

EP 3 147 366 A1

Figure 3 cont.:

b

| PCR: | IRF3 | p65 | TLR3 |
| gRNA: | IRF3 | p65 | TLR3 |

M wt 1 2 3 GFP wt 1 2 3 GFP    M wt 1 2 3 GFP 1*

c

▨ Prediction using Sanger sequencing
data (%heteroduplexed DNA)

▢ T7E1 data, Cleaved fraction=
cleaved/ (cleaved + non-cleaved)

d

▨ Prediction from T7E1 data
EE = 100 * (1- (1- cleaved fraction)$^{0.5}$)

▢ Sanger sequencing data

Figure 4:

Figure 5:

**Europäisches Patentamt**

**European Patent Office**

**Office européen des brevets**

# EUROPEAN SEARCH REPORT

Application Number

EP 15 18 6957

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NEWTON K ET AL: "A DOMINANT INTERFERING MUTANT OF FADD/MORT1 ENHANCES DELETION OF AUTOREACTIVE THYMOCYTES AND INHIBITS PROLIFERATION OF MATURE T LYMPHOCYTES", EMBO JOURNAL, OXFORD UNIVERSITY PRESS, SURREY, GB, vol. 17, no. 3, 2 February 1998 (1998-02-02), pages 706-718, XP001182164, ISSN: 0261-4189, DOI: 10.1093/EMBOJ/17.3.706 | 1,4,5, 7-14,16 | INV. C12N15/65 C07K14/705 C07K14/47 |
| Y | * page 707, column 2, paragraphs 2,3 * | 2,3,6,15 | |
| Y | L. CONG ET AL: "Multiplex Genome Engineering Using CRISPR/Cas Systems", SCIENCE, vol. 339, no. 6121, 14 February 2013 (2013-02-14), pages 819-823, XP055236048, US ISSN: 0036-8075, DOI: 10.1126/science.1231143 | 2,3,6,15 | |
| A | * the whole document * | 1,4,5, 7-14,16 | TECHNICAL FIELDS SEARCHED (IPC)  C12N C07K |
| A | LAVALLEE T M ET AL: "2-Methoxyestradiol up-regulates death receptor 5 and induces apoptosis through activation of the extrinsic pathway", CANCER RESEARCH, AMERICAN ASSOCIATION FOR CANCER RESEARCH, US, vol. 63, no. 2, 15 January 2003 (2003-01-15), pages 468-475, XP002329788, ISSN: 0008-5472 * the whole document * | 1-16 | |

-/--

The present search report has been drawn up for all claims

| Place of search | Date of completion of the search | Examiner |
|---|---|---|
| Munich | 12 January 2016 | Seroz, Thierry |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 18 6957

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUEBER ANNE-ODILE ET AL: "Transgenic overexpression of a dominant negative mutant of FADD that, although counterselected during tumor progression, cooperates in L-myc-induced tumorigenesis.", 10 November 2004 (2004-11-10), INTERNATIONAL JOURNAL OF CANCER. JOURNAL INTERNATIONAL DU CANCER 10 NOV 2004, VOL. 112, NR. 3, PAGE(S) 536 - 540, XP002752807, ISSN: 0020-7136 * the whole document * | 1-16 | |
| A | LEI S. QI ET AL: "Repurposing CRISPR as an RNA-Guided Platform for Sequence-Specific Control of Gene Expression", CELL, vol. 152, no. 5, 1 February 2013 (2013-02-01), pages 1173-1183, XP055068548, ISSN: 0092-8674, DOI: 10.1016/j.cell.2013.02.022 * the whole document * | 1-16 | |

TECHNICAL FIELDS SEARCHED (IPC)

The present search report has been drawn up for all claims

| Place of search | Date of completion of the search | Examiner |
|---|---|---|
| Munich | 12 January 2016 | Seroz, Thierry |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9208796 A

- WO 9428143 A

**Non-patent literature cited in the description**

- **QI L S ; LARSON M H ; GILBERT L A ; DOUDNA J A ; WEISSMAN J S ; ARKIN A P ; LIM W A.** Repurposing CRISPR as an RNA-guided platform for sequence-specific control of gene expression. *Cell,* 2013, vol. 152 (5), 1173-83
- **PATRICK D. HSU ; ERIC S. LANDER ; FENG ZHANG.** Development and Applications of CRISPR-Cas9 for Genome Engineering. *Cell,* 05 June 2014, vol. 157 (6), 1262-1278
- **FERRETTI J. J. ; MCSHAN W.M. ; AJDIC D.J. ; SAVIC D.J. ; SAVIC G. ; LYON K. ; PRIMEAUX C ; SEZATE S. ; SUVOROV A.N. ; KENTON S.** Complete genome sequence of an MI strain of Streptococcus pyogenes. *Proc. Natl. Acad. Sci. U.S.A.,* 2001, vol. 98, 4658-4663
- **DELTCHEVA E. ; CHYLINSKI K. ; SHARMA CM. ; GONZALES K. ; CHAO Y. ; PIRZADA Z.A. ; ECKERT M.R. ; VOGEL J. ; CHARPENTIER E.** CRISPR RNA maturation by trans-encoded small RNA and host factor RNase III. *Nature,* 2011, vol. 471, 602-607
- **JINEK M. ; CHYLINSKI K. ; FONFARA I. ; HAUER M. ; DOUDNA J.A. ; CHARPENTIER E.** A programmable dual-RNA-guided DNA endonuclease in adaptive bacterial immunity. *Science,* 2012, vol. 337, 816-821
- **CONG, L. et al.** Multiplex genome engineering using CRISPR/Cas systems. *Science,* 2013, vol. 339, 819-823

- **MALI, P. et al.** RNA-guided human genome engineering via Cas9. *Science,* 2013, vol. 339, 823-826
- **HWANG, W.Y. et al.** Efficient genome editing in zebrafish using a CRISPR-Cas system. *Nature biotechnology,* 2013, vol. 31, 227-229
- **JINEK, M. et al.** RNA-programmed genome editing in human cells. *eLife,* 2013, vol. 2, 00471
- **DICARLO, J.E. et al.** Genome engineering in Saccharomyces cerevisiae using CRISPR-Cas systems. *Nucleic acids research,* 2013
- **JIANG, W. et al.** RNA-guided editing of bacterial genomes using CRISPR- Cas systems. *Nature biotechnology,* 2013, vol. 31, 233-239
- **GARCIA-BUSTOS et al.** *Biochim. Biophys. Acta,* 1991, vol. 1071, 83-101
- **SHALEM et al.** *Science,* 2014, vol. 343 (6166), 84-87
- **SAMULSKI RJ ; CHANG LS ; SHENK T.** A recombinant plasmid from which an infectious adeno-associated virus genome can be excised in vitro and its use to study viral replication. *J Virol,* 1987, vol. 61 (10), 3096-3101
- **WALCZAK H ; MILLER RE ; ARIAIL K ; GLINIAK B ; GRIFFITH TS ; KUBIN M ; CHIN W ; JONES J ; WOODWARD A ; LE T et al.** Tumoricidal activity of tumor necrosis factor-related apoptosis-inducing ligand in vivo. *Nature Medicine,* 1999, vol. 5 (2), 157-163

## SEQUENCE LISTING

<110> Deutsches Krebsforschungszentrum Stiftung des öffentlichen Rechts

<120> DEATH RECEPTOR BASED SELECTION MARKER

<130> D31445EP

<160> 8

<170> PatentIn version 3.5

<210> 1
<211> 18
<212> PRT
<213> Thosea asigna virus

<400> 1

Glu Gly Arg Gly Ser Leu Leu Thr Cys Gly Asp Val Glu Glu Asn Pro
1               5                   10                  15

Gly Pro

<210> 2
<211> 22
<212> PRT
<213> foot-and-mouth disease virus

<400> 2

Val Lys Gln Thr Leu Asn Phe Asp Leu Leu Lys Leu Ala Gly Asp Val
1               5                   10                  15

Glu Ser Asn Pro Gly Pro
            20

<210> 3
<211> 19
<212> PRT
<213> porcine teschovirus-1

<400> 3

Ala Thr Asn Phe Ser Leu Leu Lys Gln Ala Gly Asp Val Glu Glu Asn
1               5                   10                  15

Pro Gly Pro

<210> 4
<211> 26
<212> PRT
<213> artificial

15

<220>
<223>   NLS

<400>   4

Met Gly Thr Pro His Leu Gln Gly Phe Leu Leu Leu Phe Pro Leu Leu
1                   5                   10                  15

Leu Arg Leu His Gly Ala Ser Ala Gly Ser
            20                  25


<210>   5
<211>   23
<212>   PRT
<213>   artificial

<220>
<223>   NLS

<400>   5

Met Ala Arg Arg Leu Trp Ile Leu Ser Leu Leu Ala Val Thr Leu Thr
1                   5                   10                  15

Val Ala Leu Ala Ala Leu Glu
            20


<210>   6
<211>   8
<212>   PRT
<213>   artificial

<220>
<223>   FlagTag

<400>   6

Asp Tyr Lys Asp Asp Asp Asp Lys
1                   5


<210>   7
<211>   47
<212>   PRT
<213>   artificial

<220>
<223>   leucin-zipper

<400>   7

Pro Ser Gln Lys Ser Lys Arg Arg Thr Ser Ser Asp Arg Met Lys Gln
1                   5                   10                  15

Ile Glu Asp Lys Ile Glu Glu Ile Leu Ser Lys Ile Tyr His Ile Glu
            20                  25                  30

16

```
Asn Glu Ile Ala Arg Ile Lys Lys Leu Ile Gly Glu Arg Thr Arg
        35                  40                  45


<210>   8
<211>   6
<212>   PRT
<213>   artificial

<220>
<223>   linker

<400>   8

Ser Gly Gly Ser Ser Gly
1               5
```